# EUROPEAN PATENT SPECIFICATION

(11) **EP 1 889 856 B1**
(45) Date of publication and mention of the grant of the patent: **09.03.2011**
(21) Application number: 07113396.1
(22) Date of filing: 30.07.2007
(51) Int. Cl.: C08F 220/38

(54) **Inverse emulsions useful as thickeners for cosmetics**
Umgekehrte Emulsionen als Verdickungsmittel für Kosmetika
Émulsions inverses utiles en tant qu'épaississant pour cosmétiques

(30) Priority: 03.08.2006 IT VA20060049
(43) Date of publication of application: 20.02.2008
(73) Proprietor: Lamberti Spa, 21041 Albizzate (IT)
(72) Inventor: Mitarotonda, Andrea, 21041, Albizzate (VA) (IT); Benetti, Arianna, 21013, Gallarate (VA) (IT); Li Bassi, Giuseppe, 21026, Gavirate (VA) (IT)

(56) References cited:
- WO-A-2004/113393

## Description

The present invention relates to inverse emulsions useful as thickeners in cosmetic formulations and to the procedure for their preparation.

Particularly the inverse emulsions of the present invention comprise a crosslinked polymer obtained by polymerisation of: acrylic and/or methacrylic acid, 2-acrylamido-2-methylpropanesulfonic acid and a cationic monomer.

The inverse emulsions of the invention beside possessing high skin and hair compatibility and exhibiting good thickening properties and stability over time, show high thickening power not only in aqueous solutions, but also in oil and water emulsions; these properties makes them particularly suited for the preparation of cosmetic formulations.

With the expression "cosmetic formulations" we mean the products normally used for personal care, such as body and face creams, hair gels and lotions, hair colouring and bleaching creams, sunscreen compositions, make-up products, cleansing, moisturizing and perspiring fluids and other products for similar applications.

It is known that a technical problem often encountered in the cosmetic industry is to obtain high viscous formulations (pastes, gels) stable over time and exhibiting high compatibility with skin and hair.

By cosmetic product with high compatibility with skin and hair we mean a product that is easily absorbed through a keratinous substrate while making changes in the touch, in moisturisation and perspiration, and improving the general sensorial characteristics without altering the physiological pH.

A further essential characteristic of the thickeners employed in cosmetic formulations is that they manifest their thickening capability without negatively altering the other properties of the formulations.

It is moreover highly desirable in the cosmetic field to have thickeners in the form of stable emulsion that are able to give stable cosmetic formulations.

With the expression "stable emulsion" we mean an emulsion that in the normal storing conditions (from -10°C to 40°C) and for the usual lifetime (180-360 days) does not show phase separation, sediment, formation of floating pellicles and lumps.

With the expression "stable cosmetic product" we mean a cosmetic formulation that in the above said conditions and lifetime does not show phase separation, sediment, formation of floating pellicles and lumps.

In the specialised literature many methods are reported to regulate the rheological properties of different formulations, often including the use of polymers in the form of inverse emulsion (an inverse emulsion is an emulsion containing both an oil-in-water emulsifier and a water-in-oil emulsifier, wherein the aqueous phase is dispersed in the organic phase in very small drops), but the synthetic thickeners for cosmetics of the present invention are never described.

We cite as an example:
- EP 503853, wherein an inverse emulsion containing a polymer comprising units deriving from acrylamide, 2-acrylamido-2-methylpropanesulfonic acid and a polyfunctional monomer is described; a disadvantage of the inverse emulsions of EP 503853 is the fact that they contain traces of acrylamide, a toxic substance which is unacceptable by the present European legislative trend;
- US 6,375,959 and US 6,197,287 wherein a procedure for the preparation of cross-linked or branched polyelectrolytes based on strongly acidic monomers and other monomers, but not acrylamide, in the form of an inverse emulsion, is described;
- US 6,329,483, wherein copolymers of carboxylic acids and quaternary ammonium compounds and the preparation of gels and emulsions containing the same is described;
- US 2001/0023284, wherein copolymers of a neutral monomer (N-alkylacrylamide) with one or more monomers selected among cationic monomers, monomers bearing strongly acidic functional groups and monomers bearing weakly acidic functional groups are described;
- WO 2004/112293 wherein the Applicant describes inverse emulsions comprising a polymer obtained by polymerization of two or more anionic acrylic monomers, at least one of which comprising a strongly acid functionality (and specifically a sulfonic functional group), and of at least one cationic monomer, in definite proportions.

It is a fundamental object of the present invention an inverse emulsion useful for the preparation of cosmetic formulations wherein the weight ratio between the aqueous phase and the organic phase is from 4:1 to 2:1 and containing from 20 to 70% by weight of an acrylic polymer comprising monomeric units deriving from:
i) 2-acrylamido-2-methylpropanesulfonic acid;
ii) at least one cationic acrylic monomer of formula I: 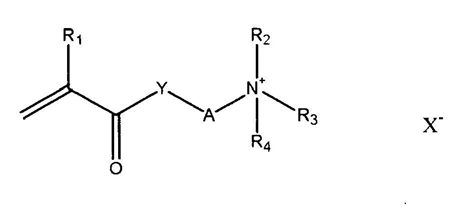 wherein R₁ is hydrogen or methyl; R₂, R₃, R₄ are, one independently of the others, hydrogen or C₁-C₄ alkyl; Y is NH or O; A is a C₁-C₆ alkylene; X is chloride;
iii) acrylic or methacrylic acid;
iv) at least one polyfunctional monomer,
the inverse emulsion being characterized by the fact that in the polymer the monomeric units deriving from 2-acrylamido-2-methylpropanesulfonic acid represent from 1% to 29% molar of the total monomers i), ii) and iii).

It is a further object of the present invention a procedure for the preparation of an inverse emulsion for cosmetic formulations characterised by: a. preparing a composition consisting of from 40 to 60% by weight of water, and for the remaining percentage by weight of a mixture of acrylic monomers consisting of i) 2-acrylamido-2-methylpropanesulfonic acid in acid form, or partially or totally in the form of salt; ii) at least one cationic acrylic monomer of formula l:

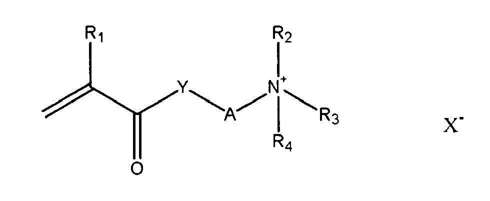

wherein R₁ is hydrogen or methyl; R₂, R₃, R₄ are, one independently of the others, hydrogen or C₁-C₄ alkyl; Y is NH or O; A is a C₁-C₆ alkylene; X is chloride; iii) acrylic or methacrylic acid

the mixture being characterized by the fact that 2-acrylamido-2-methylpropanesulfonic acid represents from 1% to 29% molar of the total monomers i), ii) and iii);

b. adding to the mixture an alkali to regulate the pH between 4 and 7;

c. adding to the mixture prepared in b. from 0.1 to 10 mmoli of a polyfunctional monomer per mole of mixture of monomers i), ii) and iii) and an initiator of radical polymerisation, maintaining the temperature between 3 and 7°C;

d. preparing an organic phase containing one or more water-in-oil emulsifiers;

e. introducing the mixture obtained in b. into the organic phase prepared in c. and emulsifying the two phases by vigorous stirring;

f. initiating the polymerisation and completing it maintaining the temperature between 55 and 95°C under vigorous stirring;

g. cooling the reaction mixture to 35-45°C and adding an oil-in-water emulsifier.

The cationic acrylic monomer of formula l is present in the acrylic polymer of the invention in a molar percentage comprised between 0.1 and 10% on the total sum of monomers i), ii) and iii) and is preferably chosen between acryloyloxyethyl-trimethylammonium chloride and methacryloyloxyethyl-trimethylammonium chloride.

The polyfunctional monomer contains two or more unsaturated reactive groups and it is present in the acrylic polymer in an amount comprised between 0.01 and 1 mmoles per mole of mixture of monomers i), ii) and iii); preferably the polyfunctional monomer is methylenebisacrylamide.

In the procedure according to the invention it is preferred to use the sodium salt of 2-acrylamido-2-methylpropanesulfonic acid, and to regulate the pH of phase b. with aqueous sodium hydroxide (NaOH).

Among the initiators of radical polymerisation utilisable for the realisation of the present invention are ammonium, potassium or sodium persulfate, and water-soluble organic peroxides, by way of example hydrogen peroxide and peracetic acid.

In the inverse emulsions of the invention the organic phase consists of mineral oils containing saturated hydrocarbons or by vegetable oils or by mixture thereof having boiling point from 150 to 300°C.

Preferably the organic phase is mineral oil, polydecene, isohexadecane, or a C₁₃-C₁₆ isopraffin; more preferably it is C₁₃-C₁₆ isoparaffin.

The water-in-oil and the oil-in-water emulsifiers are those normally used for this purpose.

We cite among the utilisable water-in-oil emulsifiers: sorbitan monolaurate, sorbitan monopalmitate, sorbitan monostearate, sorbitan monooleate; among the utilisable oil-in-water emulsifiers we cite the linear or branched ethoxylated alcohols.

The total amount of emulsifiers in the inverse emulsion of the invention is from 2 to 10% by weight; the ratio between water-in-oil emulsifiers and oil-in-water emulsifiers may range between 2:1 and 1:2.

To initiate the polymerization of the acrylic monomers an aqueous solution of sodium bisulfite may advantageously be used.

The inverse emulsions of the invention may further additionally contain the common additives used in radical polymerisation, by way of example sequestering agents such as sodium diethylenetriaminepentaacetate .

As it was previously observed, the inverse emulsions of the present invention are particularly suited for the treatment of hair and skin, in body and face creams, hair gels and lotions, hair colouring and bleaching creams, sunscreen compositions, make-up products, cleansing, moisturizing and perspiring fluids.

In the following examples the preparation of inverse emulsions according to the invention and of an inverse emulsion according to the prior art, prepared with % of 2-acrylamido-2-methylpropanesulfonic acid higher than 29% (according to WO 2004/113393), are reported, together with some application tests showing the very high thickening power of the emulsions of the present invention, in aqueous solution, in glycols, and in emulsified water and oil systems.

The stability of water and oil emulsionated systems comprising the inverse emulsions of the invention was also evaluated; the stability is correlated to the stability of the cosmetic products containing the emulsions of the invention as thickeners.

The examples illustrate the present invention without limiting it, nor the kind of application of the inverse emulsions of the invention.

Example 1.

The following ingredients are loaded into a 1.5 l pirex reactor equipped with a steel anchor stirrer:

45.5 g of de-mineralized water;

445.5 g of an 50% by weight aqueous solution of sodium 2-acrylamido-2-methylpropane sulfonate (0.972 mol);

179.9 g (2.499 mol) of acrylic acid;

0.78 g (0.003 mol) of ADAMQUAT MC 80 (acryloyloxyethyl-trimethyl ammonium chloride sold by Atofina).

After a cooling down period, necessary to reach a temperature close to 0°C, the following ingredient are slowly added while stirring:

149.8 g of a 50% aqueous NaOH solution

10 g aqueous solution (1% by weight) of methylenebisacrylamide;

0.5 g of a solution (40% by weight) of sodium diethylenetriaminepentaacetate;

11.9 g of a solution (4% by weight) of ammonium persulfate.

In the meantime, the organic phase is prepared inside a 500 ml beaker adding under stirring:

20 g of sorbitan monooleate;

232.5 g of C₁₃-C₂₀ isoparaffin

The aqueous phase is slowly added into the organic phase and subsequently the mixture is efficiently stirred with a high shear dispersing machine (ultra-turrax IKA).

The emulsion obtained is then reloaded in the reactor and the reaction is ready to be started (reaction phase). The first operation is to insufflate nitrogen directly in the bulk of the product for about 10 minutes. This is a key step, because it enables to lower and control the amount of oxygen dissolved in the emulsion and to adjust the induction times. The second phase takes place only after the emulsion temperature is warmed up to 20°C. After that, 23.9 g of a 1% by weight aqueous solution of sodium metabisulfite is quickly loaded drop-wise through an addition funnel. The third phase is the radical reaction. The reaction proceeds spontaneously raising gradually the temperature to about 60 °C in 50 minutes. The stirring is maintained very fast and cool water re-circulates inside the reactor jacket. After this period of time the emulsion is heated to 60°C and maintained at this temperature for about one hour to complete the monomers conversion, consuming the residual monomers. Subsequently a cooling down period is required to reach a temperature of 35-40°C. The final step is the addition of 25 g of C₁₂-C₁₆ linear alcohol 8 moles ethoxylated.

The mixture is rapidly stirred till homogeneity is reached; the final emulsion (Emulsion 1) is then unloaded and stored for at least 24 hours before the evaluation of its properties.

Exemple 2

The following ingredients are loaded into a 1.5 l pirex reactor equipped with a steel anchor stirrer:

45.5 g of de-mineralized water;

445.5 g of an 50% by weight aqueous solution of sodium 2-acrylamido-2-methylpropane sulfonate (0.972 mol);

179.9 g (2.499 mol) of acrylic acid;

0.78 g (0.003 mol) of ADAMQUAT MC 80 (acryloyloxyethyl-trimethyl ammonium chloride sold by Atofina).

After a cooling down period, necessary to reach a temperature close to 0°C, the following ingredient are slowly added while stirring:

149.8 g of a 50% aqueous NaOH solution

10 g aqueous solution (1% by weight) of methylenebisacrylamide;

0.5 g of a solution (40% by weight) of sodium diethylenetriaminepentaacetate;

11.9 g of a solution (4% by weight) of ammonium persulfate.

In the meantime, the organic phase is prepared inside a 500 ml beaker adding under stirring:

20 g of sorbitan monooleate;

232.5 g of C₁₆ isoparaffin

The aqueous phase is slowly added into the organic phase and subsequently the mixture is efficiently stirred with a high shear dispersing machine (ultra-turrax IKA).

The emulsion obtained is then reloaded in the reactor and the reaction is ready to be started (reaction phase). The first operation is to insufflate nitrogen directly in the bulk of the product for about 10 minutes. This is a key step, because it enables to lower and control the amount of oxygen dissolved in the emulsion and to adjust the induction times. The second phase takes place only after the emulsion temperature is warmed up to 20°C. After that, 23.9 g of a 1% by weight aqueous solution of sodium metabisulfite is quickly loaded drop-wise through an addition funnel. The third phase is the radical reaction. The reaction proceeds spontaneously raising gradually the temperature to about 60 °C in 50 minutes. The stirring is maintained very fast and cool water re-circulates inside the reactor jacket. After this period of time the emulsion is heated to 60°C and maintained at this temperature for about one hour to complete the monomers conversion, consuming the residual monomers. Subsequently a cooling down period is required to reach a temperature of 35-40°C. The final step is the addition of 25 g of C₁₂-C₁₆ linear alcohol 8 moles ethoxylated.

The mixture is rapidly stirred till homogeneity is reached; the final emulsion (Emulsion 2) is then unloaded and stored for at least 24 hours before the evaluation of its properties.

Example 3

The following ingredients are loaded into a 1.5 l pirex reactor equipped with a steel anchor stirrer:

45.5 g of de-mineralized water;

445.5 g of an 50% by weight aqueous solution of sodium 2-acrylamido-2-methylpropane sulfonate (0.972 mol);

179.9 g (2.499 mol) of acrylic acid;

0.78 g (0.003 mol) of ADAMQUAT MC 80 (acryloyloxyethyl-trimethyl ammonium chloride sold by Atofina).

After a cooling down period, necessary to reach a temperature close to 0°C, the following ingredient are slowly added while stirring:

149.8 g of a 50% aqueous NaOH solution

10 g aqueous solution (1% by weight) of methylenebisacrylamide;

0.5 g of a solution (40% by weight) of sodium diethylenetriaminepentaacetate;

11.9 g of a solution (4% by weight) of ammonium persulfate.

In the meantime, the organic phase is prepared inside a 500 ml beaker adding under stirring:

20 g of sorbitan monooleate;

232.5 g of C₂₀ hydrogenated polydecene

The aqueous phase is slowly added into the organic phase and subsequently the mixture is efficiently stirred with a high shear dispersing machine (ultra-turrax IKA).

The emulsion obtained is then reloaded in the reactor and the reaction is ready to be started (reaction phase). The first operation is to insufflate nitrogen directly in the bulk of the product for about 10 minutes. This is a key step, because it enables to lower and control the amount of oxygen dissolved in the emulsion and to adjust the induction times. The second phase takes place only after the emulsion temperature is warmed up to 20°C. After that, 23.9 g of a 1% by weight aqueous solution of sodium metabisulfite is quickly loaded drop-wise through an addition funnel. The third phase is the radical reaction. The reaction proceeds spontaneously raising gradually the temperature to about 60 °C in 50 minutes. The stirring is maintained very fast and cool water re-circulates inside the reactor jacket. After this period of time the emulsion is heated to 60°C and maintained at this temperature for about one hour to complete the monomers conversion, consuming the residual monomers. Subsequently a cooling down period is required to reach a temperature of 35-40°C. The final step is the addition of 25 g of C₁₂-C₁₆ linear alcohol 8 moles ethoxylated.

The mixture is rapidly stirred till homogeneity is reached; the final emulsion (Emulsion 3) is then unloaded and stored for at least 24 hours before the evaluation of its properties.

Example 4 (comparative)

The following ingredients are loaded into a 1.5 l pirex reactor equipped with a steel anchor stirrer:

62.21 g of de-mineralized water;

573 g of an 50% by weight aqueous solution of sodium 2-acrylamido-2-methylpropane sulfonate 1.24 mol);

135 g (1.875 mol) of acrylic acid;

0.53 g (0.003 mol) of ADAMQUAT MC 80 (acryloyloxyethyl-trimethyl ammonium chloride sold by Atofina).

After a cooling down period, necessary to reach a temperature close to 0°C, the following ingredient are slowly added while stirring:

112.38 g of a 50% aqueous NaOH solution

10 g aqueous solution (1% by weight) of methylenebisacrylamide;

0.5 g of a solution (40% by weight) of sodium diethylenetriaminepentaacetate;

10.75 g of a solution (4% by weight) of ammonium persulfate.

In the meantime, the organic phase is prepared inside a 500 ml beaker adding under stirring:

20 g of sorbitan monooleate;

214.8 g of C₁₃-C₂₀ isoparaffin

The aqueous phase is slowly added into the organic phase and subsequently the mixture is efficiently stirred with a high shear dispersing machine (ultra-turrax IKA).

The emulsion obtained is then reloaded in the reactor and the reaction is ready to be started (reaction phase). The first operation is to insufflate nitrogen directly in the bulk of the product for about 10 minutes. This is a key step, because it enables to lower and control the amount of oxygen dissolved in the emulsion and to adjust the induction times. The second phase takes place only after the emulsion temperature is warmed up to 20°C. After that, 21.5 g of a 1% by weight aqueous solution of sodium metabisulfite is quickly loaded drop-wise through an addition funnel. The third phase is the radical reaction. The reaction proceeds spontaneously raising gradually the temperature to about 60 °C in 50 minutes. The stirring is maintained very fast and cool water re-circulates inside the reactor jacket. After this period of time the emulsion is heated to 60°C and maintained at this temperature for about one hour to complete the monomers conversion, consuming the residual monomers. Subsequently a cooling down period is required to reach a temperature of 35-40°C. The final step is the addition of 25 g of C₁₂-C₁₆ linear alcohol 8 moles ethoxylated.

The mixture is rapidly stirred till homogeneity is reached; the final emulsion (Emulsion 4) is then unloaded and stored for at least 24 hours before the evaluation of its properties.

Property evaluation of Emulsions 1-4

5 oil/water emulsions are prepared with each of the Emulsions 1 and 4 (columns E1 and E4 in the table), having the following composition:

Water 78%

Oil phase 20%

Emulsion 1 or 4 2%

Where the oil phase is respectively C12-C15 alkylbenzoate, mineral oil, cyclopentasiloxane, sunflower oil and ethylhexyl palmitate.

The stability of the formulation is evaluated with the separation test in the centrifuge, at 25°C and 6000 rpm.

In the following table (Table 1) the percentages of separation after centrifugation are shown.

**Table 1**

| | Phase separation % | |
|---|---|---|
| Oil phase: | E1 | E4* |
| C12-C15 alkyl benzoate | 0 | 0 |
| Ethylhexyl palmitate | 1 | 1 |
| Sunflower oil | 0 | 0 |
| Mineral oil | 3 | 3 |
| cyclopentasiloxane | 0 | 0 |

| | | |
|---|---|---|
| *comparative | | |

The thickening properties of the Emulsions 1 and 4 are instead evaluated by measuring their Brookfield viscosity at 2% in water, 25°C and 5 rpm as well as the viscosity of the formulations of Table 1, in the same conditions.

The obtained results are reported in Table 2.

**Table 2**

| | Viscosity mPa.s | |
|---|---|---|
| Oil phase: | E1 | E4* |
| none(aqueous solution) | 52080 | 29840 |
| C12-C15 alkyl benzoate | 88000 | 56480 |
| Ethylhexyl palmitate | 68000 | 60960 |
| Sunflower oil | 80600 | 56640 |
| Mineral oil | 55040 | 85000 |
| cyclopentasiloxane | 56800 | 88000 |

| | | |
|---|---|---|
| *comparative | | |

The thickening properties of the Emulsions 1 and 4 are also evaluated in glycerine and monopropylene glycol by measuring their Brookfield viscosity at 2%, 25°C and 5 rpm in the said solvents.

The results are reported in Table 3.

**Table 3**

| | Viscosity mPa.s | |
|---|---|---|
| solvent | E1 | E4* |
| glycerine | 87800 | 58200 |
| propylene glycol | 56200 | 43200 |

| | | |
|---|---|---|
| *comparative | | |

It is observed that the Emulsion 1, according to the invention, has better thickening properties than the Emulsion of the prior art, both in water, and in glycols, and in emulsionated water and oil systems

## Claims

1. Inverse emulsion wherein the weight ratio between the aqueous phase and the organic phase is from 4:1 to 2:1 and containing from 20 to 70% by weight of an acrylic polymer comprising monomeric units deriving from: i) 2-acrylamido-2-methylpropanesulfonic acid; ii) at least one cationic acrylic monomer of formula I : 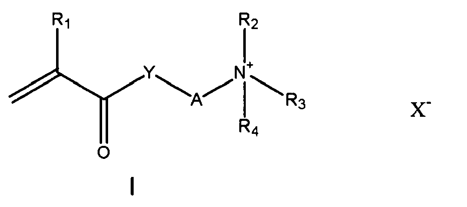 wherein R₁ is hydrogen or methyl; R₂, R₃, R₄ are, one independently of the others, hydrogen or C₁-C₄ alkyl; Y is NH or O; A is a C₁-C₆ alkylene; X is chloride; iii) acrylic or methacrylic acid; iv) between 0.01 and 1 mmoles per mole of mixture of monomers i), ii) and iii) of at least one polyfunctional monomer containing two or more unsaturated reactive groups, the inverse emulsion being **characterized by** the fact that in the polymer the monomeric units deriving from 2-acrylamido-2-methylpropanesulfonic acid represent from 1% to 29% molar of the total monomers i), ii) and iii).

2. Inverse emulsion according to claim 1., wherein cationic acrylic monomer of formula I is present in the acrylic polymer of the invention in a molar percentage comprised between 0.1 and 10% on the total monomers i), ii) and iii) and is preferably chosen between acryloyloxyethyl-trimethylammonium chloride and methacryloyloxyethyl-trimethylammonium chloride.

3. Inverse emulsion according to claims 1. or 2., wherein the polyfunctional monomer is methylenebisacrylamide.

4. Inverse emulsion according to claim 1., wherein the organic phase consists of mineral oils containing saturated hydrocarbons or by vegetable oils or by mixture thereof having boiling point from 150 to 300°C.

5. Inverse emulsion according to claim 4., wherein the organic phase is mineral oil, polydecene, isohexadecane, or a C₁₃-C₁₆ isopraffin.

6. Inverse emulsion according to claim 5., wherein the organic phase is a C₁₃-C₁₆ isoparaffin.

7. Inverse emulsion according to claim 6., containing A) a water-in-oil emulsifier selected from sorbitan monolaurate, sorbitan monopalmitate, sorbitan monostearate, sorbitan monooleate; B) as an oil-in-water emulsifier a linear or branched ethoxylated alcohol, the inverse emulsion containing a total quantity of emulsifiers comprised between 2 and 10% by weight and the weight ratio between water-in-oil emulsifiers type and oil-in-water emulsifiers ranging between 2:1 and 1:2.

8. Procedure for the preparation of an inverse emulsion for cosmetic formulations **characterised by**: a. preparing a mixture consisting of from 40 to 60% by weight of water, and for the remaining percentage by weight of a mixture of acrylic monomers consisting of: i) 2-acrylamido-2-methylpropanesulfonic acid in acid form, or partially or totally in the form of salt; ii) at least a cationic acrylic monomer of formula I 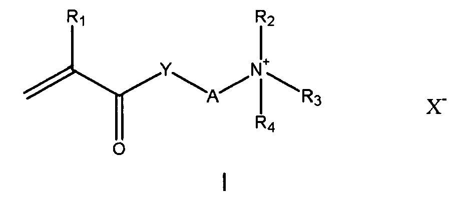 wherein R₁ is hydrogen or methyl; R₂, R₃, R₄ are, one independently of the others, hydrogen or C₁-C₄ alkyl; Y is NH or O; A is a C₁-C₆ alkylene; X is chloride; iii) acrylic or methacrylic acid, the mixture being **characterized by** the fact that 2-acrylamido-2-methylpropanesulfonic acid represent from 1% to 29% molar of the total monomers i), ii) and iii); b. adding to the composition an alkali to regulate the pH between 4 and 7; c. adding to the composition prepared in b. from 0.1 to 10 mmoles of a polyfunctional monomer containing two or more unsaturated reactive groups per mole of mixture of monomers i), ii) and iii) and an initiator of radical polymerisation, maintaining the temperature between 3 and 7°C; d. preparing an organic phase having boiling point from 150 to 300°C and consisting of mineral oils containing saturated hydrocarbons, vegetable oils or mixture thereof containing one or more water-in-oil emulsifiers; e.
introducing the mixture obtained in c. into the organic phase prepared in d. and emulsifying the two phases by vigorous stirring; f. initiating the polymerisation and completing it maintaining the temperature between 55 and 95°C under vigorous stirring; g. cooling the reaction mixture to 35-45°C and adding an oil-in-water emulsifier.

9. Procedure for the preparation of an inverse emulsion according to claim 8., wherein 2-acrylamido-2-methylpropanesulfonic acid in the form of sodium salt is used and the pH is regulated in phase b. with aqueous sodium hydroxide (NaOH).

## Patentansprüche

1. Umkehremulsion, worin das Gewichtsverhältnis zwischen wässriger Phase und organischer Phase zwischen 4:1 und 2:1 liegt und die zwischen 20 und 70% Gewichtsanteile eines Acrylpolymers enthält_{;} dessen Monomereinheiten sich aus folgenden Verbindungen herleiten: i) 2-Acrylamido-2-methylpropansulfonsäure: ii) mindestens einem kationischen Acrylmonomer der Formel (I) 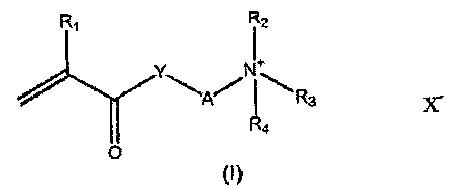 worin R₁ Wasserstoff oder Methyl ist; R₂, R₃, R₄ jeweils voneinander unabhängig Wasserstoff oder C₁-C₄ Alkyle sind; Y ein NH oder O ist; A ein C₁-C₆ Alkylen ist; X ein Chlorid ist; iii) Acryl- oder Methacrylsäure; iv) 0.01 bis 1 mmol pro Mol der Monomermischung i), ii) und iii) von mindestens einem polyfunktionalen Monomer, der zwei oder mehr ungesättigte reaktive Gruppen enthält, wobei die Umkehremulsion **dadurch gekennzeichnet ist, dass** im Polymer die aus der 2-Acrylamido-2-methylpropansulfonsäure stammenden Monomereinheiten 1% bis 29% Mol der Gesamtmonomere i), ii) und iii) darstellen.

2. Umkehremulsion gemäß Patentanspruch 1., worin das kationische Acrylmonomer der Formel (I) im erfindungsgemäßen Acrylpolymer in einem Molprozentsatz von 0.1 bis 10% der Gesamtmonomere i), ii), und iii) vorhanden ist und vorzugsweise aus Acryloyloxyethyl-trimethylammoniumchlorid und Methacryloyloxyethyltrimethylammoniumchlorid gewählt wird.

3. Umkehremulsion gemäß Patentanspruch 1. oder 2., worin das polyfunktionale Monomer Methylenbisacrylamid ist.

4. Umkehremulsion gemäß Patentanspruch 1. worin die organische Phase aus gesättigte Kohlenwasserstoffe enthaltenden Mineralölen oder aus Pflanzenölen oder aus einer Mischung der beiden besteht und einen Siedepunkt zwischen 150 und 300°C aufweist.

5. Umkehremulsion gemäß Patentanspruch 4, worin die organische Phase ein Mineralöl, Polydecen, Isohexadecan oder C₁₃-C₁₆ Isoparaffin ist.

6. Umkehremulsion gemäß Patentanspruch 5., worin die organische Phase ein C₁₃-C₁₆ Isoparaffin ist.

7. Umkehremulsion gemäß Patentanspruch 6, die A) einen aus Sorbitanmonolaureat, Sorbitanmonopalmitat, Sorbitanmonostearat, Sorbitanmonooleat gewählten Wasser-in-Öl-Emulgator, B) als Öl-in-Wasser-Emulgator einen linearen oder verzweigten ethoxylierten Alkohol enthält, wobei die Umkehremulsion eine Gesamtemulgatormenge von 2 bis 10% Gewichtsanteilen enthält und das Gewichtsverhältnis zwischen Wasser-in-Öl-Emulgatoren und Öl-in-Wasser-Emulgatoren zwischen 2:1 und 1:2 liegt.

8. Verfahren zur Zubereitung einer Umkehremulsion für kosmetische Rezepturen, **gekennzeichnet durch**: a. die Zubereitung einer Mischung, die aus 40 bis 60% Gewichtsanteilen Wasser und für den restlichen prozentualen Gewichtsanteil aus einer Mischung aus Acrylmonomeren besteht, die sich wie folgt zusammensetzt: i) 2-Acrylamido-2-methylpropansulfonsäure in Säureform oder teilweise oder vollständig in Salzform; ii) mindestens ein kationisches Acrylmonomer der Formel (1) 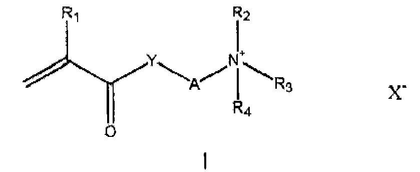 worin R₁ Wasserstoff oder Methyl ist; R₂, R₃, R₄ jeweils voneinander unabhängig Wasserstoff oder C₁-C₄ Alkyle sind; Y ein NH oder O ist: A ein C₁-C₆ Alkylen ist; X ein Chlorid ist; iii) Acryl- oder Methacrylsäure, wobei die Mischung **dadurch gekennzeichnet ist**, dass 2-Acrylamido-2-methylpropansulfonsäure 1% bis 29% Mol der Gesamtmonomere i), ii) und iii) darstellt; b. das Hinzufügen von Alkali zu der Mischung, um den pH zwischen 4 und 7 zu regulieren, c. das Hinzufügen zu der in b. gewonnenen Mischung von 0.1 bis 10 mmol eines polyfunktionalen Monomers, der zwei oder mehr ungesättigte reaktive Gruppen pro Mol der Monomermischung i), ii) und iii) enthält und eines Radikalpolymerisationsinitiators, unter Aufrechterhaltung der Temperatur zwischen 3 and 7°C; d. die Zubereitung einer organischen Phase, deren Siedepunkt zwischen 150 und 300°C liegt und die aus gesättigten Kohlenwasserstoffen, Pflanzenölen oder einer Mischung der beiden besteht und einen oder mehrere Wasser-in-Öl-Emulgatoren enthält; e.
das Hinzufügen der in c. erhaltenen Mischung zu der in d. zubereiteten organischen Phase und Emulgierung der beiden Phasen **durch** energisches Rühren; f. die Initiation der Polymerisation und deren Vollendung unter Aufrechterhaltung der Temperatur zwischen 55 und 95°C bei energischem Rühren.

9. Verfahren zur Zubereitung einer Umkehremulsion gemäß Patentanspruch 8, worin 2-Acrylamido-2-methylpropansulfonsäure in Natriumsalzform verwendet wird und der pH in Phase b. mit wässrigem Natriumhydroxid (NaOH) geregelt wird.

## Revendications

1. Émulsion inverse dans laquelle le rapport massique entre la phase aqueuse et la phase organique va de 4:1 à 2:1 et contenant de 20 à 70 % en poids d'un polymère acrylique comprenant des motifs monomères dérivés de i) l'acide 2-acrylamido-2-méthylpropanesulfonique et ii) au moins un monomère acrylique cationique de formule I : 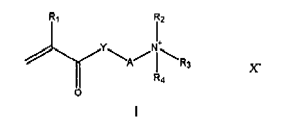 dans laquelle R₁ est un atome d'hydrogène ou un groupe méthyle ; R₂, R₃, R₄ sont chacun, indépendamment les uns des autres, un atome d'hydrogène ou un groupe alkyle en C₁ à C₄ ; Y est un groupe NH ou un atome d'oxygène ; A est un groupe alkylène en C₁ à C₆ ; X est un ion chlorure ; iii) de l'acide acrylique ou méthacrylique ; iv) entre 0,01 et 1 mmoles par mole de mélange de monomères i), ii) et iii) d'au moins un monomère polyfonctionnel contenant deux groupes réactifs insaturés ou plus, l'émulsion inverse étant **caractérisée en ce que** dans le polymère, les motifs monomères dérivés de l'acide 2-acrylamido-2méthylpropanesulfonique représentent de 1 % en mole à 29 % en mole de la totalité des monomères i), ii) et iii).

2. Émulsion inverse selon la revendication 1, ledit monomère acrylique cationique de formule (I) étant présent dans le polymère acrylique de l'invention dans un pourcentage molaire compris entre 0,1 et 10 % de la totalité des monomères i), ii) et iii), et étant de préférence choisi entre le chlorure d'acryloyloxyéthyltriméthylammonium et le chlorure de méthacryloyloxyéthyltriméthylammonium.

3. Émulsion inverse selon la revendication 1 ou 2, le monomère polyfonctionnel étant le méthylène-bis-acrylamide.

4. Émulsion inverse selon la revendication 1, la phase organique consistant en des huiles minérales contenant des hydrocarbures saturés ou en des huiles végétales ou en un mélange de ces deux types d'huiles, ayant un point d'ébullition de 150 à 300°C.

5. Émulsion inverse selon la revendication 4, la phase organique étant une huile minérale, le polydécène, l'isohexadécane ou une isoparaffine en C₁₃ à C₁₆.

6. Émulsion inverse selon la revendication 5, la phase organique étant une isoparaffine en C₁₃ à C₁₆·

7. Émulsion inverse selon la revendication 6, contenant A) un émulsifiant eau-dans-huile choisi parmi le monolaurate de sorbitane, le monopalmitate de sorbitane, le monostéarate de sorbitane, le monooléate de sorbitane ; B) en tant qu'émulsifiant huile-dans-eau, un alcool éthoxylé linéaire ou ramifié, l'émulsion inverse contenant une quantité totale d'émulsifiants comprise entre 2 et 10 % en poids et le rapport massique entre les émulsifiants de type eau-dans-huile et les émulsifiants de type huile-dans-eau variant entre 2:1 et 1:2.

8. Mode opératoire de préparation d'une émulsion inverse pour formulations cosmétiques **caractérisé par** : a. la préparation d'un mélange constitué de 40 à 60 % en poids d'eau, le complément à 100 pour cent en poids étant constitué d'un mélange de monomères acryliques consistant en : i) de l'acide 2-acrylamido-2-méthylpropanesulfonique sous forme acide, ou partiellement ou totalement sous forme de sel ; ii) d'au moins un monomère acrylique cationique de formule I : 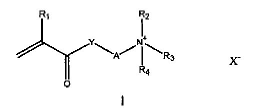 dans laquelle R₁ est un atome d'hydrogène ou un groupe méthyle ; R₂, R₃, R₄ sont chacun, indépendamment les uns des autres, un atome d'hydrogène ou un groupe alkyle en C₁ à C₄; Y est un groupe NH ou un atome d'oxygène ; A est un groupe alkylène en C₁ à C₆; X est un ion chlorure; iii) de l'acide acrylique ou méthacrylique, le mélange étant **caractérisé en ce que** l'acide 2-acrylamido-2-méthylpropanesulfonique représente de 1% en mole à 29 % en mole de la totalité des monomères i), ii) et iii) ; b. l'ajout à la composition d'un alcali afin de réguler le pH entre 4 et 7 ; c. l'ajout, dans la composition préparée en b., de 0,1 à 10 mmoles d'un monomère polyfonctionnel contenant deux groupes réactifs insaturés ou plus par mole de mélange de monomères i), ii) et iii) et d'un initiateur de polymérisation radicalaire, en maintenant la température entre 3 et 7 °C; d. la préparation d'une phase organique ayant un point d'ébullition de 150 à 300 °C et constituée d'huiles minérales contenant des hydrocarbures saturés ou en des huiles végétales ou en un mélange de ces deux types d'huiles contenant un ou plusieurs émulsifiants eau-dans-huile ; e. l'introduction du mélange obtenu en c. dans la phase organique préparée en d. et émulsion des deux phases par agitation vigoureuse ; f. l'initiation de la polymérisation et sa réalisation complète en maintenant la température entre 55 et 95 °C, sous agitation vigoureuse ; g. le refroidissement du mélange réactionnel à 35-45 °C et l'ajout d'un émulsifiant huile-dans-eau.

9. Mode opératoire de préparation d'une émulsion inverse selon la revendication 8, l'acide 2-acrylamido-2méthylpropanesulfonique étant sous forme de sel de sodium et le pH étant régulé lors de la phase (b) avec de l'hydroxyde de sodium (NaOH) aqueux.
